# EUROPEAN PATENT APPLICATION

(11) **EP 2 108 369 A1**
(43) Date of publication of application: **14.10.2009**
(21) Application number: 08425234.5
(22) Date of filing: 08.04.2008
(51) Int. Cl.: A61K 31/341, A23L 1/00, A61K 8/49, A61Q 17/00, A61P 17/16, A61P 37/04

(54) **Compounds for the treatment of immunosuppression in the skin caused by aggressive agents**

(71) Applicant: Giuliani S.p.A., 20129 Milano (IT)
(72) Inventor: Benedusi, Anna, 20124 Milano (IT); Giuliani, Giammaria, 20123 Milano (IT)
(74) Representative: Appoloni, Romano

(57) **Abstract**

The invention relates to 2,4-O-furfurylidenesorbitol or alkyl ethers thereof, for the treatment of immunosuppression in the skin caused by aggressive agents such as pollutants, dehydrating agents, ultraviolet rays, thermal and osmotic shock, and a pharmaceutical, dermatological, nutritional or cosmetic composition containing these, as is or in a mixture, as active principle.

## Description

The skin represents an effective barrier against the damaging effects of the environment, protecting the internal organs. The main layers of the skin are the epidermis, the dermis and the hypodermis. Human epidermis is relatively thick and consists of up to fifteen layers. It represents the body's first defensive barrier against harmful agents of a physical, chemical and environmental nature, for example ultraviolet rays, thermal and osmotic shock and the increasingly widespread problem of environmental pollutants.

Suppression of the immune system of the skin is known to be one of the mechanisms whereby said harmful agents cause, for example, the development of skin cancer, mainly involving the epidermal layer and the cells thereof.

For example, ultraviolet radiation causes skin alterations, both immediate and delayed in relation to the time of exposure. Besides visible alterations, such as the formation of sun burn cells, melanin production by melanocytes and thickening of the horny layer linked to the action of specific cytokeratins, some molecular alterations are also produced, which are responsible for long-term damages such as: depletion of endogenous antioxidants, which occurs in the hours immediately following exposure to the rays and which causes a cascade of time-delayed degenerative reactions (known as oxidative stress) involving the entire skin structure, epidermis and dermis; the immune reaction involving Langerhans cells and which is mediated by cytokines and other soluble factors; molecular DNA damage, in particular through the formation of 8-oxy D guanosine.

The following references in the literature regarding the effects of ultraviolet rays in terms of immunosuppression can be cited:
Katiyar S, Elmets CA, Katiyar SK. Green tea and skin cancer: photoimmunology, angiogenesis and DNA repair. J Nutr Biochem. 2007 May;18(5):287-96.
Kondo S, Jimbow K. Dose-dependent induction of IL-12 but not IL-10 from human keratinocytes after exposure to ultraviolet light A. J Cell Physiol, 1998, 177(3):493-8.
Schwarz A, Schwarz T. Molecular determinants of UV-induced immunosuppression. Exp Dermatol. 2002;11 Suppl 1:9-12.
Skiba B, Neill B, Piva TJ. Gene expression profiles of TNF-alpha, TACE, furin, IL-1beta and matrilysin in UVA- and UVB-irradiated HaCat cells. Photodermatol Photoimmunol Photomed. 2005 Aug;21 (4):173-82.
Yawalkar N, Limat A, Brand CU, Braathen LR. Constitutive expression of both subunits of interleukin-12 in human keratinocytes. J Invest Dermatol. 106(1):80-3, 1996.
Curiel-Lewandrowski C, Venna SS, Eller MS, Cruikshank W, Dougherty I, Cruz PD Jr, Gilchrest BA. Inhibition of the elicitation phase of contact hypersensitivity by thymidine dinucleotides is in part mediated by increased expression of interleukin-10 in human keratinocytes. Exp Dermatol. 2003 Apr;12(2):145-52.
Schwarz T. Mechanisms of UV-induced immunosuppression. Keio J Med. 2005 Dec; 54(4):165-71.
Haghdoost S et al. Extracellular 8-oxo dG as a sensitive parameter for oxidative stress in vivo and in vitro, Free.Rad.Res. 2005 39 (2):1 153-62.

The patent EP686026, by the same Applicant, describes the use of a combination of compounds comprising tocopherol, tolopheryl esters and 2,4-O-furfurylidenesorbitol or alkyl ethers thereof, mainly in preparations for cosmetic use to protect the skin from oxidative processes caused by UV rays, such as sun creams. Nonetheless, in general it is not possible to foresee whether in addition to this type of antioxidant activity this compound, or combination of compounds, also has immunosuppressive, and thus immunoprotective, activity of the type cited above,

The specific object of the present invention is to provide a means for effectively combating the immunosuppressive action on the skin caused by aggressive agents such as pollutants, dehydrating agents, ultraviolet rays, thermal and osmotic shock, and others.

According to the present invention it has now surprisingly been found on the basis of an experimental study that 2,4-O-furfurylidenesorbitol, or alkyl ethers thereof, are an effective active principle to combat the immunosuppressive action of these aggressive agents on the skin.

The invention therefore concerns the use of this active principle in the preparation of a pharmaceutical, dermatological, nutritional or cosmetic composition to combat the immunosuppressive action on the skin caused by aggressive agents such as pollutants, dehydrating agents, ultraviolet rays, thermal and osmotic shock, and the relative composition, characterized in that it comprises as active principle one or more compounds selected from 2,4-O-furfurylidenesorbitol or alkyl ethers thereof.

In the present description 2,4-O-furfurylidenesorbitol is also called sorbitylfurfural.

In a composition of the invention said active principle is preferably present from 0.10 to 1 p/w (%).

Among the ether alkyls thereof, 2,4-monofurfurylidene-tetra-O-methyl-sorbitol is preferred for the purposes of the invention and is the object of the experimental study described below.

In an embodiment of the invention, the composition includes compounds with antioxidant activity, such as catechin or quercetin or mixtures thereof.

The composition of the invention can be formulated for topical use or for systemic use.

Some examples of composition according to the present invention are described below. They must not be intended as limiting.

In these examples, the quantities of the components are expressed as percentage by weight within an interval.

2,4-O-furfurylidenesorbitol is defined therein as sorbityl furfural.

### Example 1

| SUN CREAM | |
|---|---|
| Components (INCl name) | Quantity p/w (%) |
| | |
| PEG- 30 DIPOLYHYDROXYSTEARATE | 2.00-5.00 |
| POLYGLYCERYL-4 DIISOSTEARATE POLYHYDROXYSTEARATE | |
| SEBACATE | 2.00-6.00 |
| BATYL ALCOHOL | 0.03- 0.080 |
| ETHYLHEXYL METHOXYCINNAMATE | 5.00- 11.000 |
| DIETHYLAMINO HIDROXYBENZOYLHEXYL BENZOATE | 5.00-11.000 |
| BUTHYL METHOXYDIBENZOYLMETHANE | 1.00-4.00 |
| ETHYLHEXYL SALICYLATE | 1.00-5.000 |
| CAPRYLIC/CAPRIC TRIGLYCERIDE | 1.00 - 10.00 |
| C12-15 ALKYL BENZOATE | 3.00-10.00 |
| BUTYLENE GLYCOL DICAPRYLATE/DICAPRATE | 4.00-10.00 |
| DIISOPROPYL SEBACATE | 1.00-5.00 |
| BUTYROSPERMUM PARKII BUTTER | 0.40-3.00 |
| ZEA MAYS OIL | 0.50-1.00 |
| PERSEA GRATISSIMA OIL UNSAPONIFIABLES | 0.40-2.00 |
| CALENDULA OFFICINALIS | 1.00-5.00 |
| TRIETHYL CITRATE | 0.20-0.80 |
| VP/EICOSENE COPOLYMER | 0.30-2.00 |
| GLYCERYL BEHENATE/ EICOSADIOATE | 1.00- 3.00 |
| PENTAERYTHRITYL TETRA-DI-T-BUTYL | |
| HYDROXYHYDROCINNAMATE | 0.01- 0.050 |
| ETHYLHEXYL TRIAZONE | 1.00-4.000 |
| MAGNESIUM STEARATE | 0.10- 0.80 |
| BETA-SITOSTEROL | 0.05-0.50 |
| BENZOIC ACID | 0.20-0.50 |
| TRICLOSAN | 0.02-0.70 |
| GLYCYRRHETINIC ACID | 0.02-0.70 |
| BORON NITRIDE | 0.20-0.50 |
| TITANIUM DIOXIDE | 0.50-5.00 |
| ALUMINUM HYDROXIDE | 0.50-5.00 |
| STEARIC ACID | 0.50-3.00 |
| GLYCERIN | 1.00- 5.00 |
| SORBITYL FURFURAL | 10-0.90 |
| CATECHIN | 0.005-0.05 |
| MAGNESIUM SULFATE | 0.70 |
| CYCLOPENTASILOXANE | 1.00-5.00 |
| CAPRYLYL GLYCOL | 0.10-0.80 |
| QUERCETIN | 0.001-0.10 |
| TETRASODIUM DICARBOXYMETHYL GLUTAMATE | 0.10-0.05 |
| PARFUM | 0.20 |
| BIS-ETHYLHEXYL HYDROXYDIMETHOXY BENZYLMALONATE | 0.01-0.50 |
| HYDROGENATED LECITHIN | 0.05-1.00 |
| WATER | q.s. 100 g |

### Example 2

| SUN CREAM | |
|---|---|
| Component (INCl name) | Qantity p/w (%) |
| | |
| C10-18 TRIGLYCERIDES | 1.00 - 10.00 |
| C12-15 ALKYL BENZOATE | 3.00- 10.00 |
| BUTYLENE GLYCOL DICAPRYLATE/DICAPRATE | 4.00-10.00 |
| DIISOPROPYL SEBACATE | 1.00-5.00 |
| BEHENYL ALCOHOL | 0.40-3.00 |
| SQUALANE | 0.40-2.00 |
| PEG- 30 DIPOLYHYDROXYSTEARATE | 2.00-5.00 |
| POLYGLYCERYL-4 DIISOSTEARATE POLYHYDROXYSTEARATE SEBACATE | 2.00-6.00 |
| BATYL ALCOHOL | 0.03- 0.08 |
| ETHYLHEXYL METHOXYCINNAMATE | 5.00- 11.00 |
| DIETHYLAMINO HIDROXYBENZOYL HEXYL BENZOATE | 5.00-11.00 |
| BUTHYL METHOXYDIBENZOYLMETHANE | 1.00-4.00 |
| ETHYLHEXYL SALICYLATE | 1.00-5.00 |
| ZEA MAYS OIL | 0.50-1.00 |
| CALENDULA OFFICINALIS | 1.00-5.00 |
| TRIETHYL CITRATE | 0.20-0.80 |
| VP/EICOSENE COPOLYMER | 0.30-2.00 |
| GLYCERYL BEHENATE/ EICOSADIOATE | 1.00- 3.00 |
| PENTAERYTHRITYL TETRA-DI-T-BUTYL HYDROXYHYDROCINNAMATE | 0.01- 0.050 |
| ETHYLHEXYL TRIAZONE | 1.00-4.000 |
| MAGNESIUM STEARATE | 0.10- 0.80 |
| B ETA-S ITOSTE ROL | 0.05-0.50 |
| BENZOIC ACID | 0.20-0.50 |
| TRICLOSAN | 0.02-0.70 |
| GLYCYRRHETINIC ACID | 0.02-0.70 |
| BORON NITRIDE | 0.20-0.50 |
| TITANIUM DIOXIDE | 0.50-5.00 |
| ALUMINUM HYDROXIDE | 0.50-5.00 |
| STEARIC ACID | 0.50-3.00 |
| GLYCERIN | 1.00- 5.00 |
| CATECHIN | 0.005-0.05 |
| MAGNESIUM SULFATE | 0.10-0.80 |
| CYCLOPENTASILOXANE | 1.00-5.00 |
| CAPRYLYL GLYCOL | 0.50-0.800 |
| SORBITYL FURFURAL | 0.10-0.90 |
| QUERCETIN | 0.001-0.1 |
| TETRASODIUM DICARBOXYMETHYL GLUTAMATE | 0.10-0.05 |
| PARFUM | 0.200 |
| COLEUS FORSKOHLII ROOT EXTRACT | 0.01-0.10 |
| BIS-ETHYLHEXYL HYDROXYDIMETHOXY BENZYLMALONATE | 0.01-0.50 |
| HYDROGENATED LECITHIN | 0.05-1.00 |
| WATER | q.s. 100 g |

### Example 3

| SUN CREAM | |
|---|---|
| Component (INCl name) ..... | Quantity p/w (%) |
| | |
| PEG- 30 DIPOLYHYDROXYSTEARATE | 1.00-5.00 |
| POLYGLYCERYL-4 DIISOSTEARATE POLYHYDROXYSTEARATE SEBACATE | 1.00-5.00 |
| BATYL ALCOHOL | 0.03- 0.08 |
| ETHYLHEXYL METHOXYCINNAMATE | 5.00-11.00 |
| BUTYL METHOXYDIBENZOYLMETHANE | 0.50-1.00 |
| DIETHYLAMINO HIDROXYBENZOYL HEXYL BENZOATE | 5.00-11.00 |
| ETHYLHEXYL SALICYLATE | 2.00-7.00 |
| CAPRYLIC/CAPRIC TRIGLYCERIDE | 3.00-8.00 |
| C12-15 ALKYL BENZOATE | 3.00-8.00 |
| BUTYLENE GLYCOL DICAPRYLATE/DICAPRATE | 3.00-8.00 |
| DIISOPROPYL SEBACATE | 1.00-6.00 |
| BUTYROSPERMUM PARKII BUTTER | 0.40-3.00 |
| PERSEA GRATISSIMA OIL UNSAPONIFIABLES | 0.40-3.00 |
| OLEA EUROPEA | 0.40-3.00 |
| CALENDULA OFFICINALIS EXTRACT | 0.40-3.00 |
| VP/EICOSENE COPOLYMER | 0.50-1.20 |
| GLYCERYL BEHENATE/ EICOSADIOATE | 2-3.500 |
| TRIETHYL CITRATE | 0.10-0.60 |
| PENTAERYTHRITYL TETRA-DI-T-BUTYL | |
| HYDROXYHYDROCINNAMATE | 0.025-0.050 |
| ETHYLHEXYL TRIAZONE | 1.00-2.500 |
| BETA-SITOSTEROL | 0.05-0.300 |
| BENZOIC ACID | 0.20-0.30 |
| TRICLOSAN | 0.20-0.500 |
| GLYCYRRHETINIC ACID | 0.020-0.500 |
| BORON NITRIDE | 0.020-0.500 |
| SORBITYL FURFURAL | 0.10-0.90 |
| GLYCERIN | 1.00-5.00 |
| MAGNESIUM SULFATE | 0.02-0.90 |
| XANTHAN GUM | 0.20-0.40 |
| CYCLOPENTASILOXANE | 1.00-3.50 |
| CAPRYLYL GLYCOL | 0.50-0.800 |
| SILICA DIMETHYL SILYLATE | 1.00-3.00 |
| ALUMINUM STARCH OCTENYLSUCCINATE | 1.00-3.00 |
| TETRASODIUM DICARBOXYMETHYL GLUTAMATE | 0.10-0.50 |
| PARFUM | 0.20 |
| COLEUS FORSKOHLII ROOT EXTRACT | 0.005-0.05 |
| BIS-ETHYLHEXYL HYDROXYDIMETHOXY BENZYLMALONATE | 0.10-0.50 |
| HYDROGENATED LECITHIN | 0.05-0.150 |
| WATER | q.s. 100 g |

### Example 4

| AFTER SUN BODY LOTION | |
|---|---|
| Component (INCl name) | Quantity p/w (%) |
| | |
| GLYCERYN | 1.00-6.00 |
| CETYL HYDROXYETHYLCELLULOSE | 0.10-0.40 |
| XANTHAN GUM | 0.10-0.40 |
| ALLANTOIN | 0.10-0.35 |
| TAPIOCA STARCH | 1.00-4.00 |
| SODIUM HYALURONATE | 0.025-0.35 |
| DISODIUM EDTA | 0.025-0.20 |
| CATECHIN | 0.005-0.05 |
| SORBITAN STEARATE | 2.00-5.00 |
| SUCROSE COCOATE | 0.10-1.00 |
| GLYCIRREHTINIC ACID | 0.30-0.70 |
| SQUALANE | 2.00-5.00 |
| BETA-SITOSTEROL | 0.10-0.50 |
| CAPRILC/CAPRIC TRIGLYCERIDES | 1.00-5.00 |
| BUTYROSPERMUM PARKII | 1.00-5.00 |
| CALENDULA OFFICINALIS | 1.00-3.00 |
| DIMETHICONE | 1.00-3.00 |
| QUERCETIN | 0.001-0.1 |
| SODIUM HYDROXIMETHYLGLYCINATE | 0-25-0.50 |
| LACTIC ACID | q.s. |
| PARFUM | 0.30 |
| DELTA TOCOPHEROL | 0.02-0.25 |
| SORBITYL FURFURAL | 0.10-0.90 |
| WATER | q.s. 100 g |

### Example 5

| AFTER SUN FACE CREAM | |
|---|---|
| Component (INCl name) | Quantity p/w (%) |
| | |
| GLYCERYN | 2.0-5.0 |
| TAPIOCA STARCH | 1.00-2.00 |
| CETYL HYDROXYETHYLCELLULOSE | 0.10-0.50 |
| SODIUM HYALURONATE | 0.05-0.5 |
| TETRASODIUM EDTA | 0.10-0.50 |
| CATECHIN | 0.005-0.05 |
| GLYCIRREHTINIC ACID | 0.10-0.70 |
| CETEARYL OLIVATE | 1.00-4.00 |
| SORBITAN OLIVATE | 0.50-3.00 |
| BUTYROSPERMUM PARKII | 1.0-8.00 |
| CETOSTEARYL ALCOHOL | 0.50-2.00 |
| BETA SITOSTEROL | 0.1050 |
| QUERCETIN | 0.001-0.1 |
| DELTA TOCOFEROL. | 0.05-0.20 |
| DIMETHICONE | 0.50-1.50 |
| DIMETHICONE CROSSPOLYMER | 0.20-1.50 |
| SORBITYL FURFURAL | 0.5-1.00 |
| CALENDULA OFFICINALIS. | 0.50-5.00 |
| SODIUM HYDROXIMETHYLGLYCINATE | 0-25-0.50 |
| PARFUM | q.s. |
| ACQUA | q.s. 100.00 |

### Example 6

| AFTER SUN MOISTURIZING GEL-CREAM | |
|---|---|
| Component (INCl name) | Quantity p/w (%) |
| | |
| GLYCERYN | 2.0-5.0 |
| POLYSORBATE 60 | 0.50-2.50 |
| SODIUM HYALURONATE | 0.05-0.5 |
| DISODIUM EDTA | 0.05-0.10 |
| GLYCIRREHTINIC ACID | 0.10-0.70 |
| ACRYLATES/C10-30 ALKYL | |
| ACRYLATE CROSSPOLYMER | 0.15-0.25 |
| SQUALANE | 1.00-2.00 |
| ETHYLHEXYL PALMITATE | 2.00-4.00 |
| CAPRILIC/CAPRIC TRIGLYCERIDES | 1.00-6.00 |
| CETOSTEARYL ALCOHOL | 0.50-2.00 |
| BETA SITOSTEROL | 0.1050 |
| DELTA TOCOFEROL | 0.05-0.20 |
| CYCLOPENTASILOXANE | 0.50-1.50 |
| SORBITYL FURFURAL | 0.5-1.00 |
| CALENDULA OFFICINALIS | 0.50-1.00 |
| SODIUM HYDROXIMETHYLGLYCINATE | 0-25-0.50 |
| SODIUM HYDROXIDE | q.s. pH 6 |
| PARFUM | q.s. |
| WATER | q.s. 100.00 |

The effectiveness of the active principle according to the invention was verified by in vitro experimentation.

This study is described below with reference to the graphs in the figures of the accompanying drawings.

Fig. 1 shows a graph indicating the expression of the cytokine IL-12.

Fig. 2 shows a graph indicating the expression of GCLC, catalytic glutamate L-cysteine ligase.

Fig. 3 shows a graph indicating the expression of Glutathione reductase.

Fig. 4 shows a graph indicating the expression of Glutathione synthetase, as described in greater detail below.

### IN VITRO STUDY

### 1. INTRODUCTION AND OBJECTIVE

A study was conducted on human epidermis reconstructed in vitro to assess both the antioxidant action and the immunoprotective and preventive action of DNA damage caused by UV rays applied to the epidermis according to the minimal erythemal dosage (dosage defined 1 MED).

The compound according to the invention specifically examined in the study described is 2,4-monofurfurylidene-tetra-O-methyl-sorbitol, compound abbreviated to ARGB11 in the graphs of the accompanying figures.

The experimental approach according to the study allows early identification, and therefore of great importance for the dermatological action of preventing photodamage, of the degree of activity of the molecule studied on the oxidative stress and immunosuppressive processes caused by UV rays, in a physiological condition of vital epidermis following UV stress using relevant, dynamic and highly sensitive parameters such as redox state, expression of mediators and molecular DNA damage.

The immunomodulating activity of some cytokines, in particular interleukin IL-12, on the immunocompetent cells, i.e. the Langerhans cells, is known. The use of this early mechanism of the immune response mediated by epidermal keratinocytes following UV irradiation was therefore hypothesized in order to assess the activity of 2,4-monofurfurylidene-tetra-O-methyl-sorbitol.

The expression of IL-12 was assessed with the technique RT-PCR. IL-12 expressed by epidermal keratinocytes after radiation attenuates the degradation processes and modulates the negative effects of UV rays, above all those responsible for DNA damage, thus playing a protective role.

Protection from DNA damage was assessed through the formation of dimers of a pyrimidine base (thymine) at the level of the basal and suprabasal epidermal layers that are already observed 5-6 hours after exposure: these are known markers of NMSC (non melanoma skin cancer) and their formation is assessed with an immunohistochemical technique.

The same technique is used to assess the formation of 8-oxy D-guanosine, specific marker of DNA damage caused by UVA.

The figures of the accompanying drawings show graphs relative to the corresponding expressions (RQ) for a non irradiated control (CTR), an irradiated control (IR) and the compound of the invention (AR GB11).

Each pair of adjacent columns in the graphs refers respectively to the case of a 4 hour UV treatment (lighter left column, 4H) and a 24 hour treatment (darker right column, 24H), irradiated according to 1 MED.

### 2. RESULTS

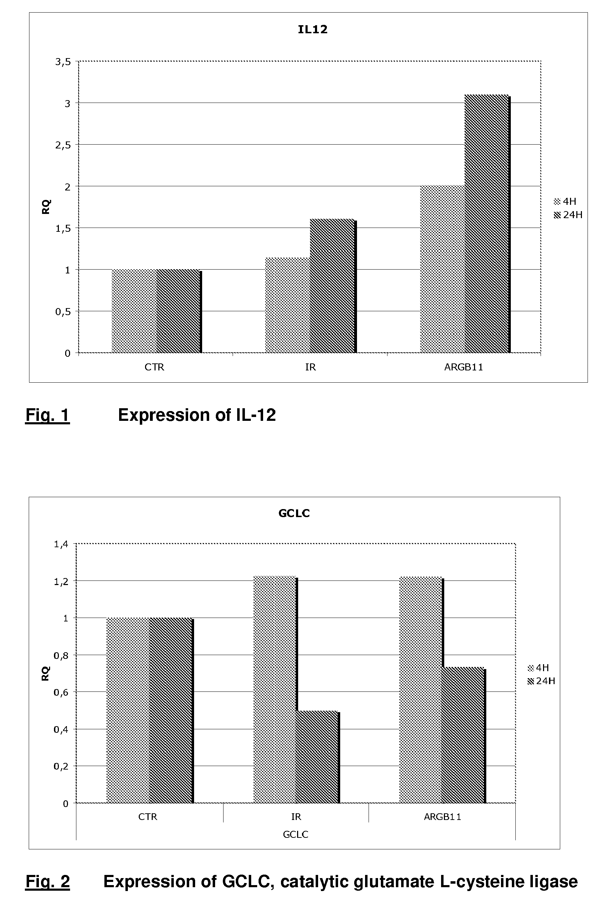

Fig. 1 presents the results on control tissues and on treated tissues relative to the expression of IL-12.

The graph shows the protective activity of the treatment with 2,4-monofurfurylidene-tetra-O-methyl-sorbitol as the gene is immunomodulated both at 4h and at 24h.

It should be observed that, after irradiation, the role of IL-12 is different (promoting DNA repair) from that hypothesized in skin sensitization mechanisms.

The results of the immunofluorescent histological assessment confirmed in relation to the 8-oxy- D-guanosine, bio-markers of UVA-induced oxidative stress at 4h from irradiation, that ARGB11 has a protective effect as the formation of 8-oxy- D-guanosine is in fact inhibited with respect to the control IR.

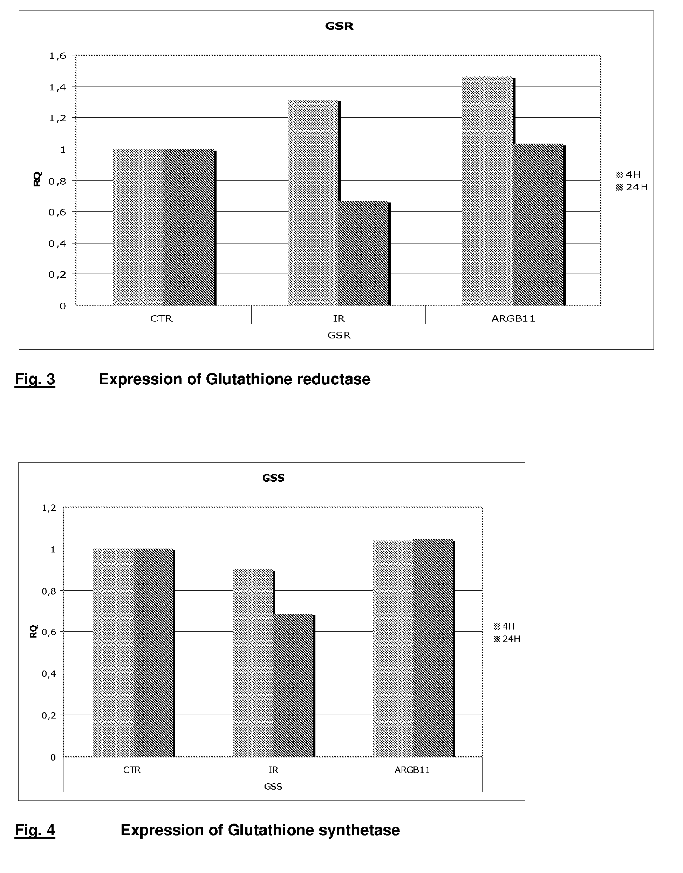

Figs. 2, 3 and 4 of the accompanying drawings present the results relative to the Glutathione GSH pathway in the control tissues and in the tissue treated for 4 and 24 hours, and precisely the expression of: catalytic glutamate L-cysteine ligase GCLC (fig. 2), Glutathione reductase GRS (fig. 3), Glutathione synthetase GSS (fig. 4). (Moreover: GPX, glutathione peroxidase; GSH, reduced glutathione; GSSG, oxidized glutathione).

GCLC is the enzyme precursor of GSH synthesis: the results of fig. 2 for the irradiated control IR at 4h show the start of the action of physiological stimulus of GSH production, while the time 24h proves to be late but significant for depletion of GSH activated by irradiation.

The results of fig. 2 indicate at the time 24h that 2,4-monofurfurylidene-tetra-O-methyl-sorbitol maintains higher levels of GCLC compared to the irradiated control IR.

Glutathione reductase GSR is the enzyme that allows the reduction of oxidized glutathione GSSG to reduced glutathione GSH, and is complementary to glutathione peroxidase G PX.

In fig. 3 it can be observed that the irradiated control tissue IR shows an overexpression at 4h, although not significant, while at 24h the decrease is significant and coherent with the mechanism it regulates: the tissue is in an oxidation state caused by irradiation.

With regard to 2,4-monofurfurylidene-tetra-O-methyl-sorbitol, the increase compared to the control of fig. 3 indicates that the treatment with this compound acts as stimulus for restoration of the physiological redox state characteristic of skin homeostasis to restore GSH to a higher level than that of GSSG.

Finally, glutathione synthetase GSS is the enzyme limiting GSH synthesis: in fig. 4 the irradiated control IR is consistently underexpressed with the oxidation state.

The results of the graph show ARGB11 activity that restores values similar to those of the non irradiated control CTR.

To summarize, the study according to the invention shows for 2,4-monofurfurylidene-tetra-O-methyl-sorbitol an expected antioxidant action on the one hand, and on the other a surprising immunoprotective action, both early and delayed, essentially on the basis:
- of the expression of glutathione reductase GSR with a stimulus for restoration of the physiological redox state characteristic of skin homeostasis to restore GSH to a higher level than that of GSSG;
- of the results toward the DNA-UVA damage caused;
- of the expression of IL-12.

The objects of the invention are therefore shown to be effectively realized.

## Claims

1. 2,4-O-furfurylidenesorbitol, or alkyl ethers thereof, for the treatment of immunosuppression in the skin caused by aggressive agents such as pollutants, dehydrating agents, ultraviolet rays, thermal and osmotic shock.

2. Use of 2,4-O-furfurylidenesorbitol or alkyl ethers thereof for immunosuppression in the skin caused by aggressive agents such as pollutants, dehydrating agents, ultraviolet rays, thermal and osmotic shock.

3. Use according to claim 2, **characterized in that** said alkyl ether is 2,4-monofurfurylidene-tetra-O-methyl-sorbitol.

4. Use according to claim 2, **characterized by** associating the use of compounds with antioxidant activity selected from catechin or quercetin or a mixture thereof.

5. Use of 2,4-O-furfurylidenesorbitol or alkyl ethers thereof for the preparation of a composition for the treatment of immunosuppression in the skin caused by aggressive agents such as pollutants, dehydrating agents, ultraviolet rays, thermal and osmotic shock.

6. Pharmaceutical, dermatological, nutritional or cosmetic composition to combat the immunosuppressive action on the skin caused by aggressive agents such as pollutants, dehydrating agents, ultraviolet rays, thermal and osmotic shock, **characterized in that** it comprises as active principle one or more compounds selected from 2,4-O-furfurylidenesorbitol or alkyl ethers thereof.

7. Composition according to claim 6, **characterized in that** said alkyl ether is 2,4-monofurfurylidene-tetra-O-methyl-sorbitol.

8. Composition according to claim 6, **characterized by** comprising compounds with antioxidant activity selected from catechin or quercetin or a mixture thereof.

9. Composition according to the preceding claims, **characterized in that** said active principle is present from 0.10 to 1 p/w (%).

10. Composition according to the preceding claims, **characterized in that** it is formulated for topical use.
